# EUROPEAN PATENT SPECIFICATION

(11) **EP 0 979 245 B1**
(45) Date of publication and mention of the grant of the patent: **17.11.2004**
(21) Application number: 98913511.6
(22) Date of filing: 27.04.1998
(51) Int. Cl.: C07K 14/81, C07K 5/10

(54) **OLIGOPEPTIDIC INHIBITORS OF ELASTASES AND METHODS OF PREPARATION THEREOF**
OLIGOPEPTIDINHIBITOREN FÜR ELASTASE UND VERFAHREN ZU DEREN HERSTELLUNG
INHIBITEURS OLIGOPEPTIDIQUES D'ELASTASES ET PROCEDES DE PREPARATION DE CES INHIBITEURS

(30) Priority: 30.04.1997 CZ 131597
(43) Date of publication of application: 16.02.2000
(73) Proprietor: THE PROCTER & GAMBLE COMPANY, Cincinnati, Ohio 45202 (US)
(72) Inventor: KASAFIREK, Evzen, 162 00 Praha 6 (CZ); PIHERA, Pavel, 108 00 Praha 10 (CZ); STURC, Antonin, 143 00 Praha 4 (CZ); SEDO, Aleksi, 155 00 Praha 5 (CZ)
(74) Representative: Veronese, Pancrazio
(86) International application number: PCT/CZ1998/000019
(87) International publication number: WO 1998/049197

(56) References cited:
- WO-A-90/04409
- US-A- 4 528 133
- KASAFIREK ET AL.: "Anionic inhibitor of pancreatic and leukocyte elastase" BIOL. CHEM. HOPPE-SEYLER, vol. 366, 1985, pages 333-343, XP002074780

## Description

### Technical Field

The invention refers to oligopeptidic inhibitors of elastases, especially leukocyte elastase (LE). Elastolytic enzymes, for instance LE or pancreatic elastase (PE) are the members of the group of serine proteinases which take part in the degradation of the natural substrate elastin; elastin creates the substantial part of protein pool of organism. In the healthy organism this destructive activity is inhibited by endogenic enzymes, for instance α₁-proteinase inhibitor (α₁-PI). In the case of insufficiency of α₁-PI of various causes (infection, nutrition, working environment, genetic factors) the autodigestion of organs essential to life by LE and PE is observed, which is the basis of origin of serious diseases as for instance acute pancreatitis, pulmonary emphysema, arthritis or gingivitis.

### Background Art

One of the possibilities how to reach homeostasis - the balance between elastase and the corresponding endogenic inhibitor (in the case of a pathogenic condition) is the substitution therapy which means the application of the exogenic natural or synthetic inhibitor of type α₁-PI. This is carried out by application of either natural (Antilysin^{R}, Trasylol^{R}, Contrykal^{R}) or synthetic α₁-PI like Nafamstat mesylate, FUT (Tori Co, Tokyo, Japan). WO-A-90/04409 relates to synthetic peptide inhibitors of elastase comprising a sequence Lys-Ala-Pro with additional other groups attached. Similarly, US-A-4528133 and publication "Anionic inhibitor of pancreatic and leukocyte elastase", Biol. Chem. Hoppe-Seyler, 366, 333-343 (1985), disclose tetrapeptide alkylamides based on sequences Glu-Ala-Ala-Pro or Asp-Ala-Ala-Pro which act as inhibitors of elastase. Recently, the inventors have suggested a synthetic potent inhibitor for the therapy of acute pancreatitis Glt-Ala₃-NHEt, Inpankin VÚFB 16834 (Gut 33, 701-706, 1992).
The serious defect of the isolated (natural) inhibitors is not only their antigenicity (α₁-PI shows the species specificity), but also the possible contamination by bovine spongioformic encephalopathy (BSE). On the contrary, the synthetic, low molecular inhibitors are lacking these unwanted side effects, are more efficient and more stable. Their chemical structure is strictly defined and characterised in the chemical and physical way.

### Disclosure of Invention

The invention relates to oligopeptidic inhibitors of elastases of general structure I. 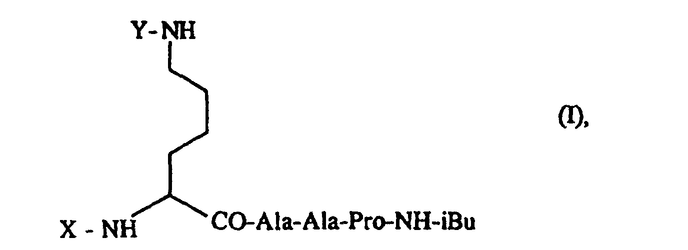 wherein
X means A or B,
Y means A or B or A-Ala, and if Y is A-Ala then X means B,
A means a saturated acid of 8 to 16 carbon atoms,
B means the 3-carboxypropionyl or 4-carboxybutyroyl residue.

According to a further aspect of the present invention there is provided a method for the preparation of compounds of general structure I wherein Y is B, characterised in contacting lysine having protected amino groups with a compound of formula Ala-Ala-Pro-NH-iBu, deprotecting the ε-amino group of the product, reacting the resulting product with a compound of formula

A-X¹,

wherein A has the above meaning and X¹ is a halogen, deprotecting the α-amino group and contacting the resulting compound of formula Lys(A)-Ala-Ala-Pro-NH-iBu with succinic or glutaric anhydride.

The invention also includes a method for the preparation of compounds of general structure I wherein Y means A, characterised in contacting a compound of formula A-Lys(W), wherein W is a protective group and A has the above meaning, with a compound of formula Lys(W), wherein W is a protective group and A has the above meaning, with a compound of formula Ala-Ala-Pro-NH-iBu, deprotecting the product and contacting the resulting compound of formula A-Lys-Ala-Ala-Pro-NH-iBu with succinic or glutaric anhydride.

The invention also includes the method for the preparation of compounds of general structure I wherein Y is A-Ala, characterised in contacting a compound of formula A-Ala with a compound of formula W-Lys-Ala-Ala-Pro-NH-iBu, wherein W is a protective group and A has the above meaning, deprotecting the product and contacting the resulting compound of formula Lys(A-Ala)-Ala-Ala-Pro-NH-iBu with succinic or glutaric anhydride.

Compounds of general structure I are efficient competitive inhibitors of serine proteinases, especially of leukocyte elastase (LE) and pancreatic elastase (PE). These enzymes are the cause of destructive damage of cell and venous walls and therefore are the cause of serious diseases. Besides the mostly observed pulmonary emphysema, acute pancreatitis and various types of arthritis, the above mentioned enzymes cause hemorrhagic periodontitis. This is the reason why one of the attractive applications of the compounds described is the use of these as the ingredients of various dental preparations, for instance dentifrices or mouth washes. An especially interesting one would be the application of these efficient inhibitors as ingredients of chewing gums; the ingredients in these medicinal preparations ensure the constant level of the active inhibitor in oral cavity also during the daily working activity, when, from the working and time reasons the standard hygienic care of the dental cavity is not possible.
Further potential use of LE inhibitors is in blocking of the activation of pro-enzymes, for instance cathepsine B (Biochim.Biophys. Acta 1226, 117-125, 1994) or for inhibition of matrix metalloproteinase 3 (MMP-3) -so called stromelysin from the corresponding zymogen (FEBS Lett. 299, 353-356, 1989). MMP-3 is known as the key enzyme in pathogenesis of various malign diseases and also in sclerosis multiplex.

The guarantee of the lack of the unwanted or toxic side effect of the inhibitors described is the fact that these are synthesised only from physiologic, so nontoxic compounds, which means aminoacids (alanine, proline, lysine), higher fatty acids and succinic or glutaric acids. Compounds of general structure I can be used in the form of free acids or (for the reason of higher solubility) in the form of soluble salts created with alkali metals, mostly with sodium. The synthesis of oligopeptidic inhibitors of elastase according to this invention including all the intermediates is described in schemes 1, 2 and 3. The final products and intermediates are characterized by their melting points (m.p.) or by their optical rotations, in some cases by thin layer chromatography (TLC). The values are mentioned in tables I-VI.
The key compound for synthesis of all oligopeptidic inhibitors is isobutylamide of alanyl-alanyl-proline (Collection Czechoslov. Chem. Commun. 52, 3034-3041, 1987); acylation of aminoacids by higher fatty residues is described in patent document No CS 280 726. The synthesis of intermediates and of final products is performed in solution and is clear from the examples of preparation. The evaluation (determination of inhibitory constants Ki) by human leukocyte elastase was performed using known procedure (Biol. Chem. Hoppe-Seyler 366, 333-343, 1985), the results of determinations of some compounds are described in table VII.

Following abbreviations and symbols were used:
- Ala =: alanine
- Pro =: proline
- Lys =: lysine
- Suc =: succinyl
- Glt =: glutaryl
- Kpl =: caproyl
- Lau =: lauroyl
- Myr =: myristoyl
- Pal =: palmitoyl
- Z =: benzyloxycarbonyl
- Boc =: tert.butyloxycarbonyl
- iBu =: isobutyl
- DCCI: N,N' dicyclohexylcarbodiimide
- OHSuc =: N-hydroxysuccinimide
- EtO-CO-Cl =: ethyl chloroformate

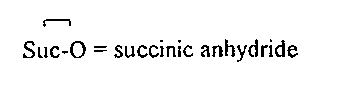

The standard preparation: the rough reaction product was solubilized in AcOEt and extracted with 1% citric acid, followed by extraction with H₂O, then with 5% NaHCO₃ and again with H₂O. The product was dried over anhydrous Na₂SO₄ and evaporated. All the evaporation was done under reduced pressure on a rotary vacuum evaporator.
Thin layer chromatography (TLC - silica-gel G) was performed in the system S₁: n-butanol-acetic acid-H₂O (4:1:1).
Optical rotation was determined using the Perkin-Elmer 141 polarimeter. Melting points were determined on the Koffler block and have not been corrected.

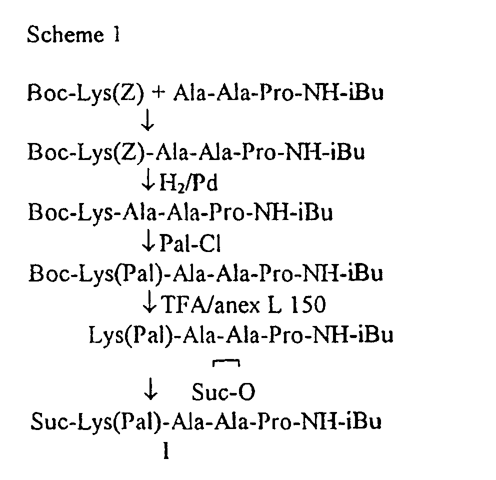

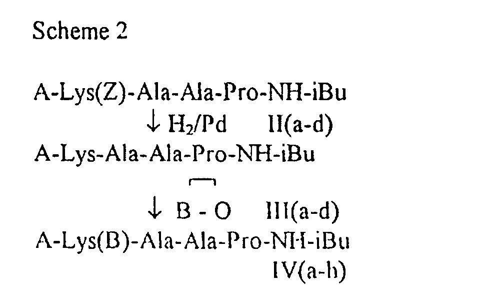

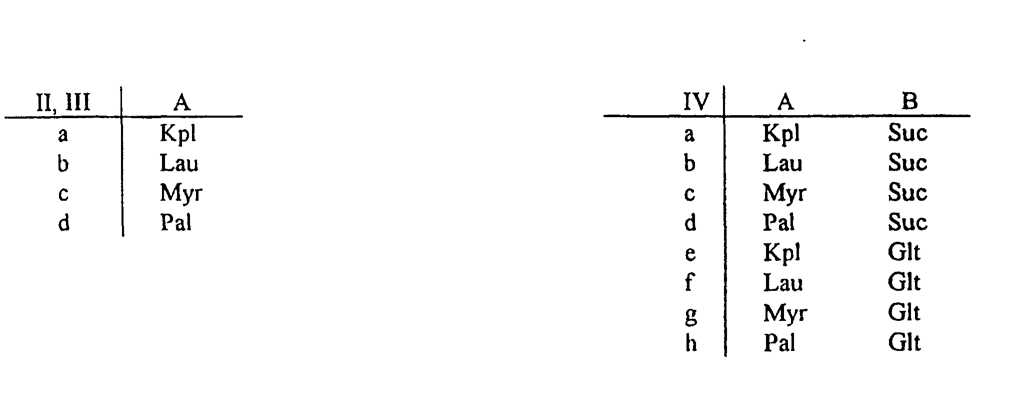

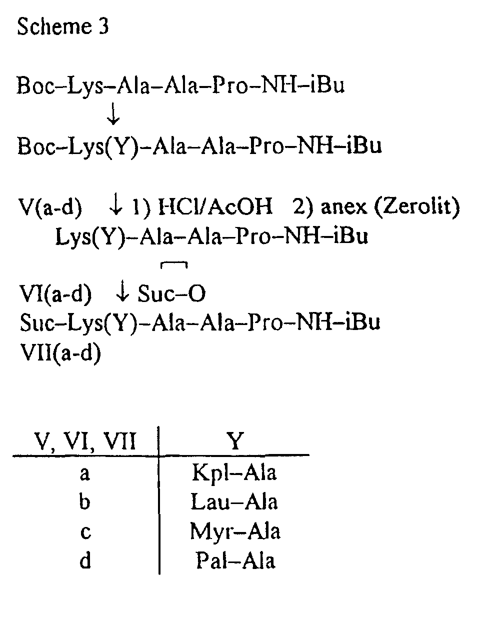

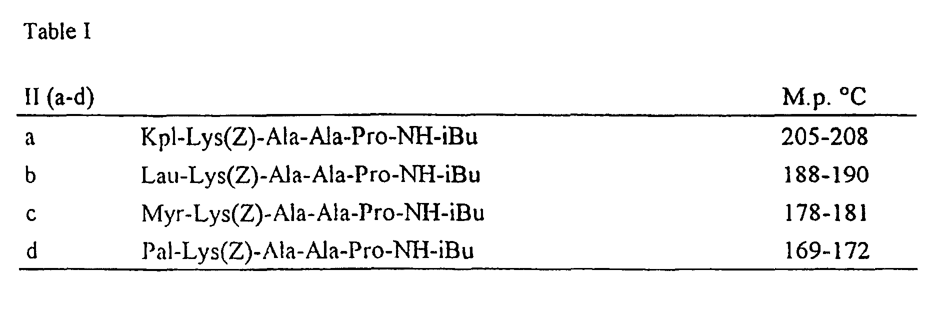

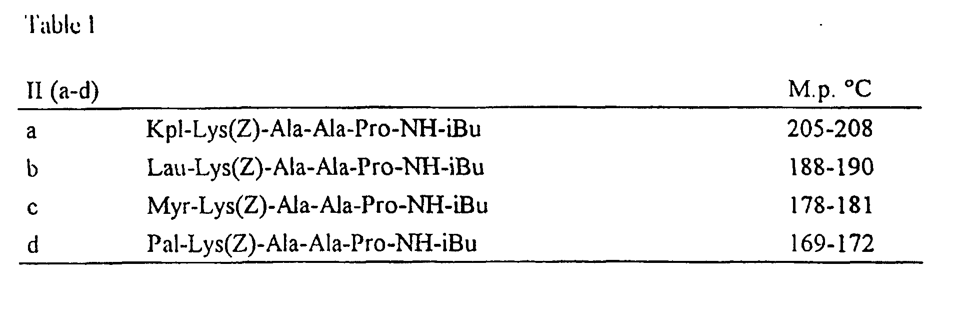

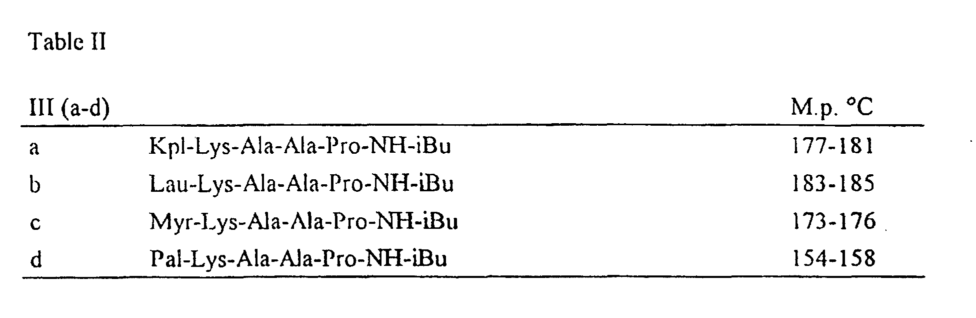

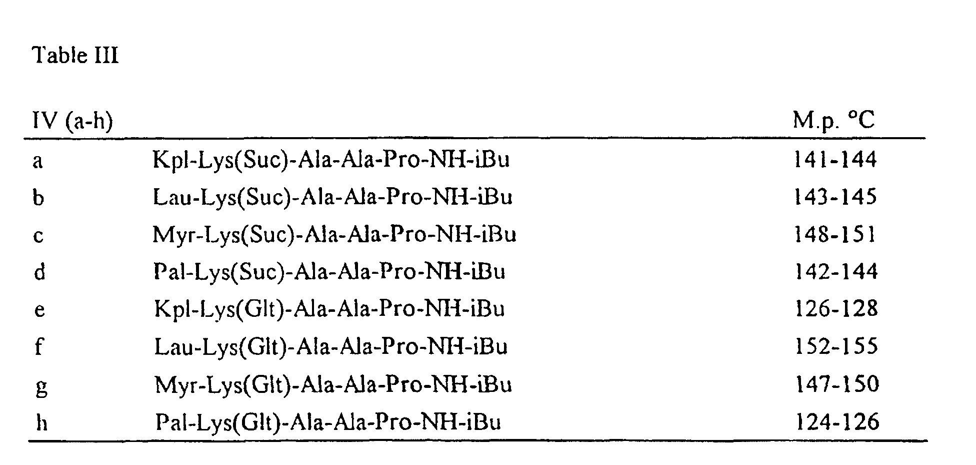

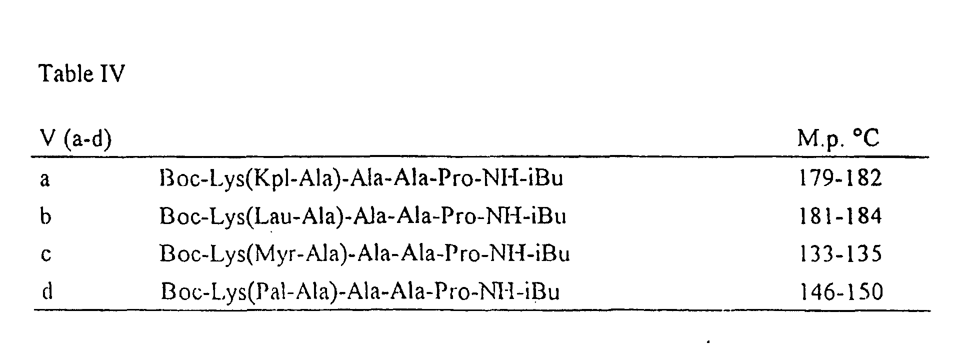

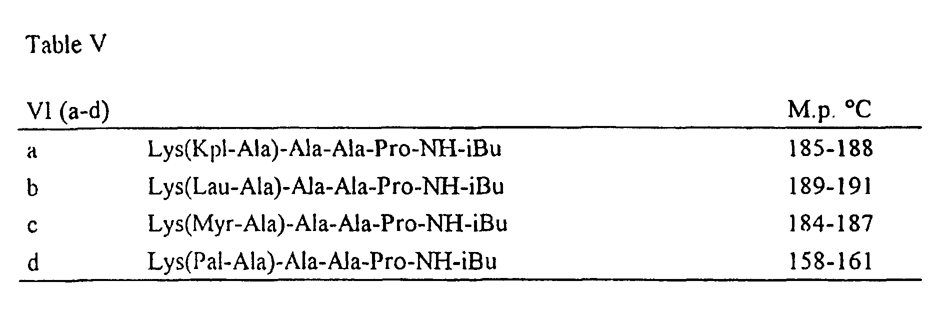

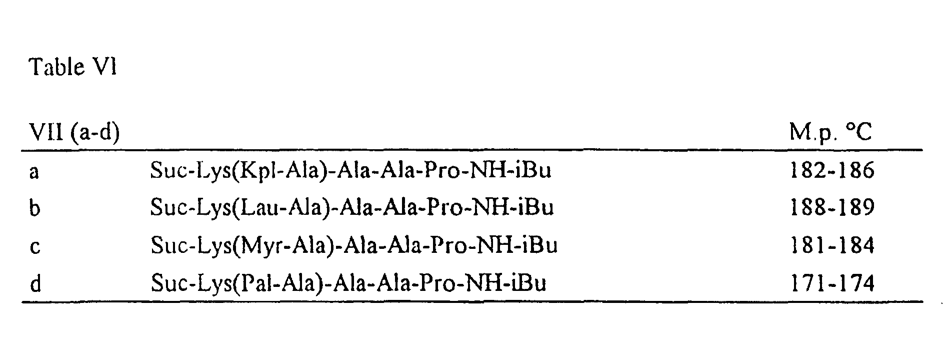

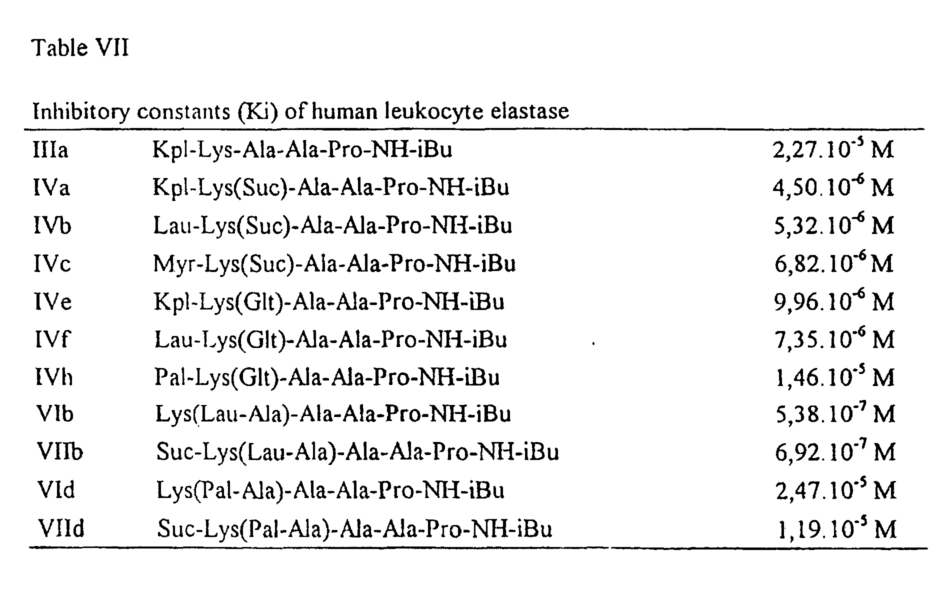

### Examples

### Example 1

### Boc-Lys(Z)-Ala-Ala-Pro-NH-iBu

To the solution of Boc-Lys(Z) (20 mmol) and N-Etp (2,8 ml) in DMFA (50 ml) cooled to -10 °C EtOCO-Cl (2 ml) was added; after 5 min stirring (-10 °C) to the reaction mixture the solution of Ala-Ala-Pro-NH-iBu (6,3 g, 20 mmol) in 20 ml DMFA was added. The mixture was stirred for 30 min (0 °C) and then the stirring continued for 2 h (room temperature). After this, the reaction mixture was evaporated, the residue solubilized in the mixture of AcOEt and H₂O and standard procedure I followed. The residue was crystallised from 2-propanol and light petroleum. 5,7 g of the product (m.p. 168-170 °C) were obtained.

### Boc-Lys-Ala-Ala-Pro-NH-iBu

To the solution of Boc-Lys(Z)-Ala-Ala-Pro-NH-iBu (3,4 g; 5 mmol) in methanol (300 ml) the suspension 5% Pd/C (0,3 g) in toluene (10 ml) was added and the reaction mixture was hydrogenated in autoclave 20 min, under the pressure 2 MPa. After removing of the catalyst by filtration, the methanol solution was evaporated. 2,8 g of product (m.p. 150-153 °C) was obtained.

### Boc-Lys(Pal)-Ala-Ala-Pro-NH-iBu

To the solution of Boc-Lys-Ala-Ala-Pro-NH-iBu (1,1 g, 2 mmol) in DMFA (4 ml) and 1 M NaOH (3,5 ml) Pal-Cl (1 ml) during 30 min was added (room temperature); after further 30 min of stirring the reaction mixture was acidified (10% AcOH, pH 5) and the crude precipitate formed was separated by filtration. 0,9 g of product (chromatographic homogeneity, R_{f}= 0,71/S₁) was obtained.

### Lys(Pal)-Ala-Ala-Pro-NH-iBu

To the solution of Boc-Lys(Z)-Ala-Ala-Pro-NH-iBu (390 mg) in AcOH (0,5 ml) TFA (0,5 ml) was added. After 1 h, when the mixture was standing at the room temperature it was evaporated and dried over P₂O₅. Then, the trifluoracetate obtained was solubilized in methanol and deionised using slightly basic anex (L 150). 180 mg of product (base, m.p. 139-142 °C; [α]²⁰_{D} -78,3° (c = 0,2; methanol) was obtained.

### Suc-Lys(Pal)-Ala-Ala-Pro-NH-iBu

To the solution of Lys(Pal)-Ala-Ala-Pro-NH-iBu (140 mg, 0,2 mmol) in DMFA (20 ml) succinic anhydride (25 mg) was added; the reaction mixture was kept at the temperature 80 °C for 30 min, then the reaction mixture was evaporated and gel residue was crystallised from the solution of DMFA and AcOEt. 60 mg of product (m.p. 157-160 °C; [α]²⁰_{D} -73,5° /c = 0,2; methanol/) was obtained.

### Example 2

### Myr-Lys(Z)-Ala-Ala-Pro-NH-iBu (IIc)

To the solution of Myr-Lys(Z) (5,05 g, 10 mmol) in DMFA (150 ml), HOSuc (1,15 g) in DMFA (20 ml) was added after cooling to 0 °C DCCI (2,2 g) was added. After 1 h stirring (0 °C) Ala-Ala-Pro-NH-iBu (3,15 g, 10 mmol) in DMFA (25 ml) was added. The reaction mixture was left to stand for 12 h at room temperature, then AcOH (0,2 ml) was added and the reaction suspension was left at 0 °C for 30 min. Then it was filtered, washed by DMFA and evaporated. The gel residue was crystallised from hot DMFA (40 ml). 4,7 g of product (m.p. 178-181 °C) was obtained. The similar procedure was used for the preparation of compounds II (a,b,d).

### Myr-Lys-Ala-Ala-Pro-NH-iBu (IIIc)

To the solution of Myr-Lys(Z)-Ala-Ala-Pro-NH-iBu (4 g; 5 mmol) in methanol (300 ml), the suspension of 5% Pd/C (0,3 g) was added the pressure hydrogenolysis was performed (2 MPa) as described in example 1. 2,9 g of product (m.p. 173-176 °C) was obtained. The similar procedure was used for the preparation of compounds III (a,b,d).

### Myr-Lys(Suc)-Ala-Ala-Pro-NH-iNH-iBu (IVc)

To the solution of Myr-Lys-Ala-Ala-Pro-NH-iBu (1,35 g, 2 mmol) in dioxane (30 ml) succinic anhydride (380 mg) was added and the reaction mixture was heated for 30 min under rellux. After cooling, 1,75 g of product (m.p. 148-151 °C) was obtained. Similar procedure was used for preparation of compounds IV (a,b,d).

### Pal-Lys(Glt)-Ala-Ala-Pro-NH-iBu (IVh)

To the solution of Pal-Lys-Ala-Ala-Pro-NH-iBu (2.05 g, 3 mmol) in dioxane (30 ml) glutaric anhydride (600 mg) was added and the reaction mixture was heated for 30 min under the reflux. After cooling 2,1 g of product was obtained (m.p. 124-126 °C; [α]²⁰_{D} - 34,6° /c = 0,2; DMFA/). Similar procedure was used for the preparation of compounds IV (e-g).

### Example 3

### Boc-Lys(Pal-Ala)-Ala-Ala-Pro-NH-iBu (Vd)

To the solution Pal-Ala (985 mg, 3 mmol) in DMFA (25 ml) OHSuc (350 mg) was added, after cooling to -5 °C DCCI (660 mg) was added, after 30 min of stirring (-5 °C) the solution of Boc-Lys-Ala-Ala-Pro-NH-iBu (1,65 g, 3 mmol) in DMFA (30 ml) was added. The mixture was stirred at room temperature for 3 h. The created precipitate was separated by filtration, washed DMFA and water (50 ml) was added. The precipitate was separated by filtration and crystallised from 2-propanol. 1,75 g of product (m.p. 146-150 °C) was obtained. The similar procedure was used for the preparation of compounds V(a-c).

### Lys(Pal-Ala)-Ala-Ala-Pro-NH-iBu (IVd)

To the solution of Boc-Lys(Pal-Ala)-Ala-Ala-Pro NH-iBu (1,7 g; 2 mmol) in AcOH (20 ml) the solution of 0,6 M HCl/AcOH (4 ml) was added. After 3 h of standing at room temperature, the created hydrochloride was precipitated by means of ether, separated by filtration and dried in the presence of P₂O₅ and NaOH. Then, the hydrochloride was deionised by strong basic anex (Zerolit FF/OH-cycle) in methanol. The solution was evaporated and the residue was crystallised from 2-propanol and light petroleum. The base (820 mg) was obtained (m.p. 158-161 °C). The similar procedure was used for the preparation of compounds VI(a-c).

### Suc-Lys(Pal-Ala)-Ala-Ala-Pro-NH-iBu (VIId)

To the solution of Lys(Pal-Ala)-Ala-Ala-Pro-NH-iBu (750 mg; I mmol) in DMFA (20 ml) succinic anhydride (160 mg) was added. After 2 h of stirring at the room temperature the product was precipitated by addition of H₂O (50 ml) and crystallised from 2-propanol and AcOEt. 690 mg of the final product was obtained (m.p. 171-174 °C). The similar procedure was used for the preparation of compounds VII(a-c).

## Claims

1. Oligopeptidic inhibitors of elastases of general structure I: 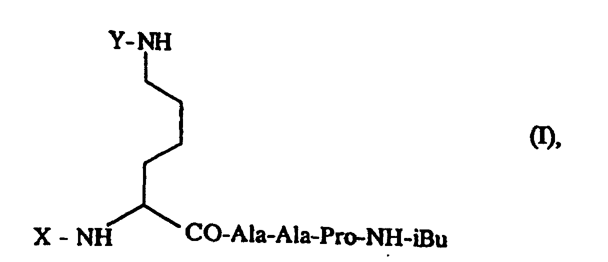 wherein
X means A or B,
Y means A or B or A-Ala, and if Y is A-Ala then X means B,
A means a saturated acid of 8 to 16 carbon atoms,
B means the 3-carboxypropionyl or 4-carboxybutyroyl residue.

2. A method for the preparation of compounds of general structure I according to claim 1, wherein Y is B, **characterized in** contacting lysine having protected amino groups with a compound of formula: Ala-Ala-Pro-NH-iBu, deprotecting the ε-amino group of the product, reacting the resulting product with a compound of formula:
A-X¹,
wherein A is defined according to claim 1 and X¹ is a halogen, deprotecting the α-amino group and contacting the resulting compound of formula Lys(A)-Ala-Ala-Pro-NH-iBu with succinic or glutar anhydride.

3. A method for the preparation of compounds of general structure I according to claim 1, wherein Y means A, **characterized in** contacting a compound of formula A-Lys(W), wherein W is a protective group and A is defined according to claim 1, with a compound of formula: Ala-Ala-Pro-NH-iBu, deprotecting the product and
contacting the resulting compound of formula A-Lys-Ala-Ala-Pro-NH-iBu with succininc or gluatric anhydride.

4. A method for the preparation of compounds of general structure I according to claim 1, wherein Y is A-Ala, **characterized in** contacting a compound of formula A-Ala with a compound of formula: W-Lys-Ala-Ala-Pro-NH-iBu, wherein W is a protective group and A is defined according to claim 1, deprotecting the product and contacting the resulting compound of formula Lys(A-Ala)-Ala-Ala-Pro-NH-iBu with succinic or glutaric anhydride.

## Patentansprüche

1. Oligopeptidische Elastaseinhibitoren der allgemeinen Struktur I: 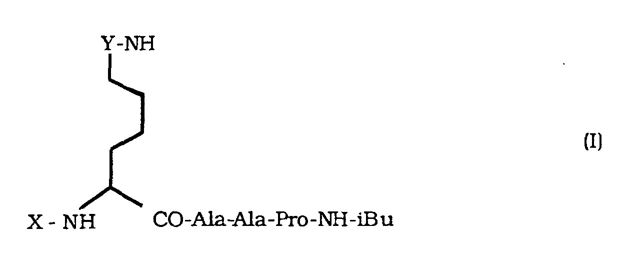 worin
X A oder B bedeutet,
Y A oder B oder A-Ala bedeutet, und wenn Y A-Ala ist, X B bedeutet,
A eine gesättigte Säure mit 8 bis 16 Kohlenstoffatomen bedeutet,
B den 3-Carboxypropionyl- oder 4-Carboxybutyroyl-Rest bedeutet.

2. Verfahren zur Herstellung von Verbindungen der allgemeinen Struktur I nach Anspruch 1, worin Y B ist, **dadurch gekennzeichnet, dass** Lysin mit geschützten Amino-Gruppen mit einer Verbindung der Formel: Ala-Ala-Pro-NH-iBu in Kontakt gebracht wird, die ε-Aminogruppe des Produkts entschützt wird, das resultierende Produkt mit einer Verbindung der Formel:
A-X¹,
worin A wie in Anspruch 1 definiert ist und X¹ ein Halogen darstellt, umgesetzt wird, die α-Aminogruppe entschützt wird und die resultierende Verbindung der Formel Lys(A)-Ala-Ala-Pro-NH-iBu mit Bernsteinsäure- oder Glutarsäureanhydrid in Kontakt gebracht wird.

3. Verfahren zur Herstellung von Verbindungen der allgemeinen Struktur I nach Anspruch 1, worin Y A bedeutet, **dadurch gekennzeichnet, dass** eine Verbindung der Formel A-Lys(W), worin W eine Schutzgruppe ist und A nach Anspruch 1 definiert ist, mit einer Verbindung der Formel: Ala-Ala-Pro-NH-iBu in Kontakt gebracht wird, das Produkt entschützt wird und die resultierende Verbindung der Formel A-Lys-Ala-Ala-Pro-NH-iBu mit Bernsteinsäure- oder Glutarsäureanhydrid in Kontakt gebracht wird.

4. Verfahren zur Herstellung von Verbindungen der allgemeinen Struktur I nach Anspruch 1, worin Y A-Ala ist, **dadurch gekennzeichnet, dass** eine Verbindung der Formel A-Ala mit einer Verbindung der Formel: W-Lys-Ala-Ala-Pro-NH-iBu, worin W eine Schutzgruppe ist und A wie in Anspruch 1 definiert ist, in Kontakt gebracht wird, das Produkt entschützt wird und die resultierende Verbindung der Formel Lys(A-Ala)-Ala-Ala-Pro-NH-iBu mit Bernsteinsäure- oder Glutarsäureanhydrid in Kontakt gebracht wird.

## Revendications

1. Inhibiteurs oligopeptidiques d'élastases, de structure générale I : 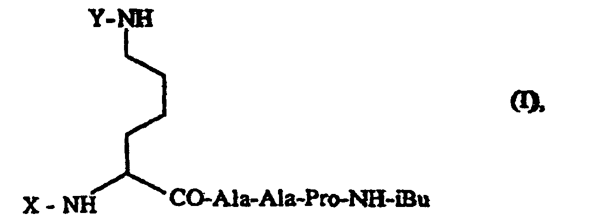 dans laquelle
X signifie A ou B,
Y signifie A ou B ou A-Ala, et si Y est A-Ala, alors X signifie B,
A signifie un acide saturé de 8 à 16 atomes de carbone,
B signifie le reste 3-carboxypropionyle ou 4-carboxybutyroyle.

2. Procédé pour la préparation de composés de structure générale I selon la revendication 1, dans lequel Y et B, **caractérisé par** la mise en contact de lysine ayant des groupes amino protégés, avec un composé de formule : Ala-Ala-Pro-NH-iBu, la déprotection du groupe ε-amino du produit, la réaction du produit obtenu avec un composé de formule :
A-X¹,
où A est défini selon la revendication 1 et X¹ est un halogène, la déprotection du groupe α-amino et la mise en contact du composé obtenu de formule Lys(A)-Ala-Ala-Pro-NH-iBu avec de l'anhydride succinique ou glutarique.

3. Procédé pour la préparation de composés de structure générale I selon la revendication 1, dans lequel Y signifie A, **caractérisé par** la mise en contact d'un composé de formule A-Lys(W), où W est un groupe protecteur et A est défini selon la revendication 1, avec un composé de formule : Ala-Ala-Pro-NH-iBu, la déprotection du produit et la mise en contact du composé obtenu de formule A-Lys-Ala-Ala-Pro-NH-iBu avec de l'anhydride succinique ou glutarique.

4. Procédé pour la préparation de composés de structure générale 1 selon la revendication 1, dans lequel Y est A-Ala, **caractérisé par** la mise en contact d'un composé de formule A-Ala avec un composé de formule : W-Lys-Ala-Ala-Pro-NH-iBu, où W est un groupe protecteur et A est défini selon la revendication 1, la déprotection du produit et la mise en contact du composé obtenu de formule Lys(A-Ala)-Ala-Ala-Pro-NH-iBu avec de l'anhydride succinique ou glutarique.
